# EUROPEAN PATENT SPECIFICATION

(11) **EP 0 309 558 B1**
(45) Date of publication and mention of the grant of the patent: **02.11.1994**
(21) Application number: 88903742.0
(22) Date of filing: 05.04.1988
(51) Int. Cl.: C12Q 1/68, G01N 33/53

(54) **METHOD FOR DETECTING THE EXPRESSION OF BIOREGULATORY GENES IN MICROCULTURES OF EUKARYOTIC CELLS**
VERFAHREN ZUR FESTSTELLUNG DER EXPRESSION BIOREGULATORISCHER GENE IN MIKROKULTUREN EUKARIONTISCHER ZELLEN
METHODE POUR DETECTION DE L'EXPRESSION DE GENES BIOREGULATEURS DANS LES MICROCULTURES DE CELLULES EUCARYOTES

(30) Priority: 15.04.1987 AU 1455/87
(43) Date of publication of application: 05.04.1989
(73) Proprietor: YISSUM RESEARCH DEVELOPMENT COMPANY OF THE HEBREW UNIVERSITY OF JERUSALEM, Surry Hills, NSW 2010 (AU)
(72) Inventor: KAEMPFER, Raymond, 93707 Jerusalem (IL); ARRAD, Gila, 96701 Jerusalem (IL); GEREZ, Lisya, 92585 Jerusalem (IL)
(74) Representative: Daley, Michael John
(86) International application number: AU8800099
(87) International publication number: WO8808038

(56) References cited:
- EP-A- 140 764
- AU-A- 8 427 546
- AU-A- 8 774 329
- US-A- 4 483 920
- NATURE, vol. 297, 20 May 1982, London (GB); S. EFRAT et al., pp. 236-239#
- METHODS IN ENZYMOLOGY, vol. 152, 1987, S.L. Berger et al. (eds.), Academic Pres, London (GB); pp. 219-226#
- ANALYTICAL BIOCHEMISTRY, vol. 137, 1984; S. CHELEY et al., pp. 15-19#
- EMBO JOURNAL, vol. 6, no. 3, 1987, IRL Press Ltd., Oxford (GB); M.A. LEBENDIKER et al., pp. 585-589#
- PROCEEDINGS OF THE NATL. ACADEMY OF SCIENCES USA, vol. 1981, May 1984; S. EFRAT et al., pp. 2601-2605#
- JOURNAL OF IMMUNOLOGY, vol. 129, no. 2, August 1982, US; M. MATSUYAMA et al., pp. 450-451#

## Description

### FIELD OF THE INVENTION

The present invention relates to a method for detecting the expression of bioregulatory genes in microcultures of eukaryotic cells. The present invention is particularly useful in diagnosis as well as gaining basic understanding of genesis and dynamics of viral, bacterial, fungal, oncogenic or immunoregulatory diseases. It is also useful for monitoring the effectiveness of therapies therefor. Diagnostic kits for carrying out the invented method are also provided.

Cytokines, soluble cell products that behave as local hormones, regulating the growth and behaviour of other cells, are involved in proper physiological functioning. Cytokines include the interferons, interleukins, tumor necrosis factors and similar substances, and the growth factors.

Study of cytokines has led to an important medical development of biotherapy. Cytokine biotherapy offers improvement over the existing treatment of cancer, viral, fungal, bacterial and immuno-regulatory diseases.

Eukaryotic cells such as lymphocytes, monocytes, fibroblasts, keratinocytes, astrocytes, endothelial cells, glial cells and platelets, are producing cytokines in response to various biological needs and functions.

In eukaryotic cells, gene expression involves the formation of messenger RNA during the process of protein synthesis. Therefore, it is possible to measure gene expression by quantitatively determining the extent of presence of mRNA specific for various cytokines.

Currently, there exists no direct method of assessing the dynamics of the immune response in a given patient, even though this information could be of great clinical importance in diagnosis and the guiding of therapy in many diseases, including cancer, autoimmune diseases and immunodeficiency syndromes. Monoclonal antibodies can be used to count subsets of T cells, but fail to report the function and dynamic responsiveness of the immune system. Three human genes are of key importance in determining the strength of the immune response. They are encoding interleukin-2 (IL-2) or T-cell growth factor, the receptor for IL-2 (IL-2R), and immune-interferon or gamma interferon (IFN-y). The expression of these genes, induced by an immune stimulus, is normally down-regulated severely (up to 99%) by novel molecular mechanisms [Efrat, S. and Kaempfer, R. Proc. Natl. Acad. Sci. USA 81, 2601-2605 (1984); Efrat, S., Zelig, S., Yagen, B. and Kaempfer, R. Biochem. Biophys Res. Commun. 123, 842-848 (1984); Kaempfer, R. and Efrat, S. Cellular and Molecular Biology of Lymphokines (C. Sorg and A. Schimpl, eds.) Academic Press, Orlando, Fla., pp. 605-618 (1985); Kaempfer, R., Efrat, S. and Marsh, S. Molecular Cloning and Analysis of Lymphokines (D.R. Webb and D. Goeddel, ets.) Academic Press, Orlando, Fla., pp. 59-72 (1987); Lebendiker, M.A., Tal, C., Sayar, D., Pilo, S. Eilon, A., Banai, Y. and Kaempfer, R. EMBO J. 6, 585-589 (1987)] elicited, in part, by interactions between subsets of cells in the immune system [Kaempfer, R., and Efrat, S. Leukocytes and Host Defense (J.J. Oppenheim and D.M. Jacobs, eds.) Alan R. Liss, Inc., NY, pp. 57-68 (1986); Lebendiker, M.A., Tal, C., Sayar, D., Pilo, S., Eilon, A., Banai, Y. and Kaempfer, R. EMBO J. 6, 585-589 (1987); Kaempfer, R., Sayar, D., Efrat, S., Lebendiker, M., Ketzinel, M. and Tal, C. Lymphokine Research 6, Vol. 1 5th International Lymphokine Workshop Abstr. 1640 (1987)]. Moreover, expression of these three genes is functionally linked, creating a network of interacting genes that are expressed in different lymphocytes.

Conventional methods for the recovery of RNA from cells generally include lysis of the cells and ultracentrifugation through a cesium chloride gradient, are time consuming, cumbersome and require large numbers of cells and heavy equipment. Procedures known to be suitable for small numbers of cells, when applied to peripheral blood cells, suffer from the deficiencies that the quantitative determination of the RNA encoded by a specific gene is not sufficiently accurate to allow precise measure to be made of gene expression.

Conventional SDS and NP-40 methods of extraction of mRNA [White, B.A., S. Bancroft F.C. J. Biol. Chem. 257:8569 (1982)] are inappropriate for the detection of cytokines because the presence of contaminating protein masks the presence of RNA and some degradation of RNA occurs during the many steps involved in these procedures.

Guanidinium thiocyanate (GCN) Direct Blot [Matsuyama M, Sugamura K, Kawade Y. and Hinuma Y.J. Immunology 129:450 (1982)] is inappropriate because the viscosity of DNA interferes with the processing of RNA fractions. This procedure has the limitation that no more than 10⁵ cells can be analyzed, too few for the detection of cytokine gene expression which is at lower levels than that of most other genes.

Other methods of recovering RNA based on guanidinium, such as GCN-LiCl (Cathala G. Savouret J.F. Mendez B. West B.L., Karin M., Martial J.A. and Baxter J.D. DNA 2:239 1983) are yielding a pure mRNA but involve too many steps and cause some loss of RNA to allow convenient processing of large numbers of samples.

A method based on guanidinium hydrochloride [Cheley S. and Anderson R. Anal. Biochem. 137:15 (1984)] has been used to detect viral RNA expressed in situations of chronic viral infections. Methods of isolating RNA using guanidinium salts, typically guanidine hydrochloride and guanidinium thiocyanate, have been disclosed [MacDonald R. J., Swift G. H., Przybyla A. E. and Chirgwin J. M. Methods in Enzymology 152:219 (1987); this reference, teaches that selective precipitations from guanidinium solutions can be nonquantitative and are affected in unforeseeable ways by components of the homogenate. This problem, it is disclosed, is generally more serious when the RNA concentration in the homogenate is very low.

The present inventors have discovered that an adaption of this method can be used for detecting the expression of bioregulatory genes in eukaryotic cells provided that appropriate steps are taken to induce expression of the bioregulatory gene or genes. The method according to this invention can be used in order to identify and quantify reliably and repeatedly the expression of bioregulatory genes in only a few millilitres of peripheral blood taken from a given patient. Further, the present inventors have discovered that by using a plurality of induction processes on separate samples of the eukaryotic cells a much greater understanding can be obtained of the dynamic functionality of the genes in the cell sample than is possible by any known technique.

Therefore, the present inventors have selected and provided a method which overcomes the shortcomings of the prior art techniques for measuring gene expression in cytokine-producing cells, inducing the expression of cytokine cells and detecting such expression in a smaller number of cells than hitherto possible, in defined culture conditions that are capable of providing measurement not only of actual expression of cytokine genes but also of the functional intactness of regulatory mechanisms that control expression of these genes and their disturbance in pathological situations. The object of the invention is to provide a highly efficient method of mRNA extraction from appropriately cultured human peripheral blood mononuclear cell populations which increases the sensitivity for detecting cytokine gene expression and avoids the pool artifacts created by the use of standard Northern blot analyses, in order to assess the normal or abnormal expression of cytokine gene and their regulation.

### SUMMARY OF THE INVENTION

The present inventors have now succeeded in providing a method that allows accurate and sensitive detection of a transient expression of bioregulatory genes in situations where such expression is usually very low and almost undetectable and where only small cell samples are available.

The technique according to this invention can, in preferred embodiments, accurately monitor the extent of gene expression in as little as half-a-million cells or up to 500-fold fewer cells than needed for the standard methods that have hitherto been used to detect the expression of such genes. This allows the study of the expression of various bioregulatory genes in only a few millilitres of peripheral blood which are easy to get from a patient and which express many genes of key bioregulatory importance.

It is an object of the present invention to provide a general method for detecting the expression of bioregulatory genes in microcultures of eukaryotic cells, having an important diagnostic and therapeutic value comprising the steps of:
(a) isolating a sample of eukaryotic cells and (b) dividing it into at least two aliquots (c) culturing the cells in each aliquot;
(d) lysing said cultured cells with guanidinium thiocyanate or guanidinium hydrochloride;
(e) homogenizing and thereafter selectively precipitating ribonucleic acid by adding to the homogenized cell preparation a sodium or potassium acetate solution followed by absolute ethanol;
(f) recovering and blotting RNA from each aliquot onto support matrix;
(g) hybridizing recovered and blotted RNA with a labelled riboprobe specific to at least one of said genes; and
(h) quantitatively determining the extent of binding of said labelled riboprobe to said blotted RNA. the method being characterised in that in step (c):
(c) (i) the cells in a first aliquot are cultured in the presence of an inducing agent for inducing expression of the bioregulatory gene; and
(c) (ii) the cells in a second aliquot are cultured in the absence of an inducing agent;
and in that:
i) the ratio of the extent of gene expression in the first aliquot to the extent of gene expression in the second aliquot is calculated, said ratio providing a measure of the inductivity of the bioregulatory gene.

In a preferred embodiment, said lysing step (b) is done by adding guanidinium thiocyanate and said selective precipitation in step (c) by addition of lithium chloride followed by sodium or potassium acetate solution and absolute ethanol.

The method according to the present invention is preferably carried out for the detection of expression of bioregulatory genes, selected from the group comprising interferons, interleukins, tumor necrosis factors, and growth factors, wherein the eukaryotic cells comprise lymphocytes, monocytes, keratinocytes, fibroblasts, astrocytes, endothelial cells, glial cells and platelets. Inducing the expression of the bioregulatory genes is preferably carried out by a compound such as phytohemagglutinin or concavalin A, with or without cycloheximide and gamma-irradiation, or their appropriate combination. Gamma-irradiation is an example of a treatment that prevents the activation of suppressor T cells [Gullberg M., Larsson E.L., J. Immunol. 128:746 (1982)], which affects IFN-y gene expression [Lebendiker, M.A., Tal C., Sayer D., Pilo S., Banai Y., Eilon A. & Kaempfer R. EMBO 6:658 (1987)]. Cycloheximide is an agent that leads to abnormal expression of the IL-2 gene [Efrat S. and Kaempfer R. Proc. Natl. Acad. Sci. USA. 81; 2601 (1984)] and the IFN-y gene [Lebendiker, M.A., Tal C., Sayar D., Pilo S., Banai Y., Eilon A. & Kaempfer R. EMBO 6:585 (1987)].

A suitable inducer is phytohemagglutinin. The ratio of the induced expression level to the basal level indicates whether gene regulation is normal or not. If desired inducement may be carried out on separate aliquots of the cell sample to provide kinetic information on the induction process.

In a further preferment according to this invention additional or alternative inducers such as cycloheximide are added to additional aliquots of the cell sample to super induce the cells. Such superinduction can indicate the maximum potential for gene expression. This in turn provides a guide as to the extent to which the basal expression level approaches the maximum potential expression of the gene.

It has been found that suppressor T cells down-regulate at least some cytokine genes. γ-irradiation prevents activation of suppressor T cells. Thus, advantageously, one aliquot of the cell-sample is subjected to superinduction and γ-irradiation. Such a sample will indicate the extent to which suppressor T cells are down-regulating expressions of the gene of interest.

The method according to this invention requires the said riboprobe to be labelled with a detectable isotopic or non-isotopic chemical group.

The invention offers convenient analysis of many samples (48 and more) without the use of heavy equipment and a rapid quantitation of data in numerical form.

The method according to this invention may be carried out conveniently and without requiring the use of an ultracentifuge, making the rapid analysis of large numbers of samples feasible; moreover, it reproduceably detects gene expression over at least a 200-fold range. The equipment needed is standard and simple in nature: microfuge, cell centrifuge, CO₂ incubator, dot-blot apparatus, waterbath.

A 10-ml sample of blood yields usually 10⁷ cells, permitting multiple analysis of gene expression (several genes, several conditions of induction). The ability to make many measurements on a single blood sample allows refinements under various conditions of induction to measure intactness of gene regulatory mechanisms in disease.

Response is linear with increasing RNA input. Response increases linearly both with cell number and amount of RNA. Three times more cells generate a signal equal to that of three times more RNA, i.e., threefold stronger. The gene expression signal is linear over at least a 200-fold range. Thus, both hybridization and film blackening are a quantitative measurement. These properties render the method highly reproducible and informative.

The product is total cell RNA and mRNA intact with no more than 5% contaminating DNA and even less protein.

There is no masking of the signal of hybridization by protein. The hybridization signal is equally as strong as that of pure RNA prepared by guanidinium thiocyanate lysis and ultracentrifugation through CsCl, a long and cumbersome procedure.

A signal of specific mRNA, e.g., for Interleukin-2 (IL-2) or Interferon-gamma (IFN-y), can be detected in as little as 5 x 10⁵ cells within 24hr of film exposure. This is 20-to-100 fold fewer cells than are needed for a standard Northern blot signal for these genes.

As used herein the units "µg" and "µl" are to be understood as meaning respectively micrograms and microlitres.

### DESCRIPTION OF THE DRAWINGS

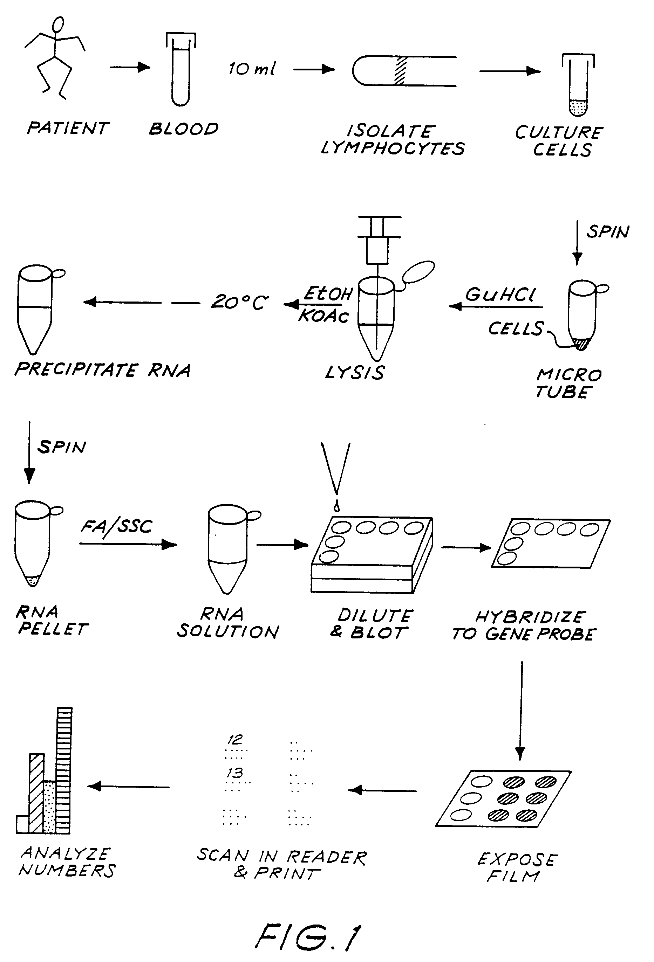

Fig. 1 is a diagrammatic outline of a procedure according to this invention;

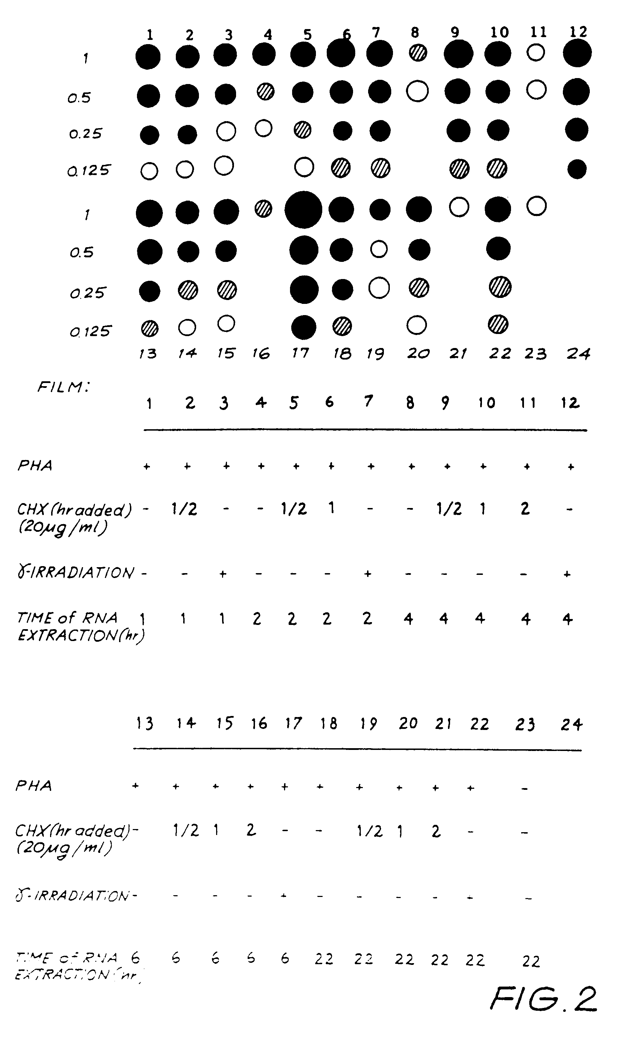

Fig. 2 demonstrates the use of the assay for measuring IFN-y gene expression. 4 x 10⁶ human peripheral blood mononuclear cells were induced for IFN-y gene expression. RNA extracted from the cells was analyzed in four serial dilutions (0.125, 0.25, 0.5 and 1) using a manifold dot-blot device. The nitrocellulose filter was hybridized to IFN-y riboprobe.
   Gamma-irradiation (γ-irradiation) was done before addition of 0.4% (v/v) phytohemagglutinin (PHA), the dose was 1500 rad. Cycloheximide (CHX) was added to several cultures after the addition of PHA. The filter was exposed to film after hybridization and the autoradiogram is shown.

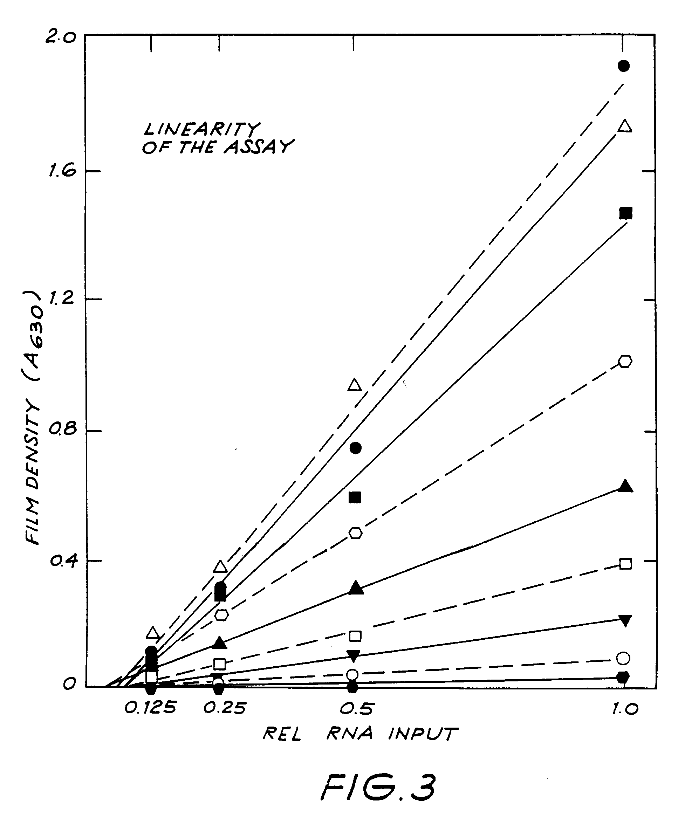

Fig. 3 is a graph showing the linearity of the assay according to this invention. Samples expressing different levels of IL-2 RNA or IFN-y RNA were analyzed in four serial dilutions (0.125, 0.25, 0.5 and 1.0) using a manifold dot-blot device. After hybridization to specific riboprobes and autoradiography of the nitrocellulose filters, absorbency of individual dots at 630nm was measured in a micro-elisa autoreader.

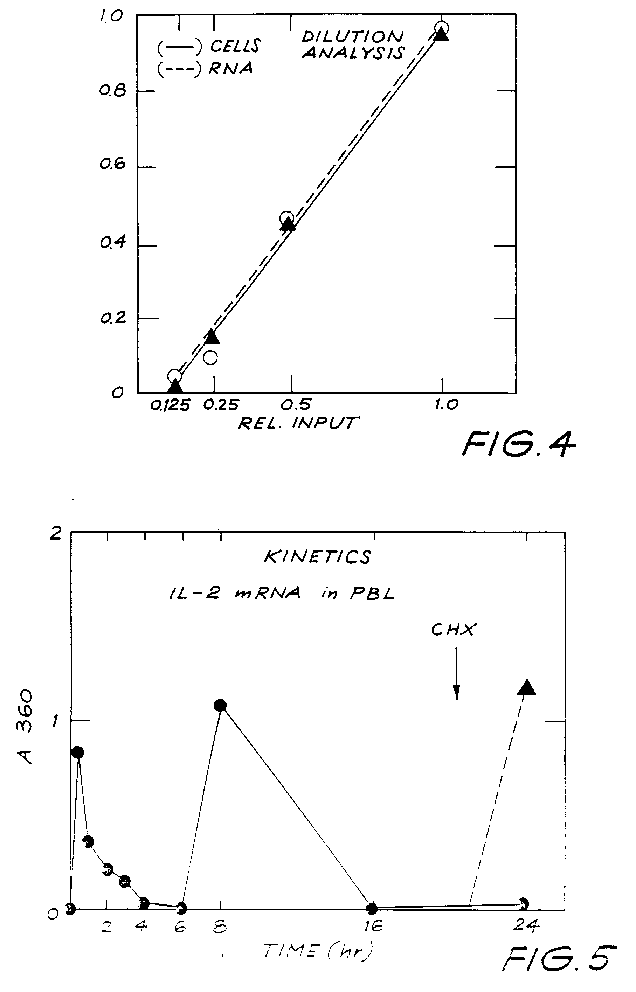

Fig. 4 is a graph showing the linearity and equivalent response of the assay as a function of increasing cell number or increasing RNA input. 4 x 10⁶ cells, induced for IL-2 gene expression, were serially diluted and RNA was then extracted (▲). Alternatively, RNA was extracted from 4 x 10⁶ cells, and then serially diluted (o).

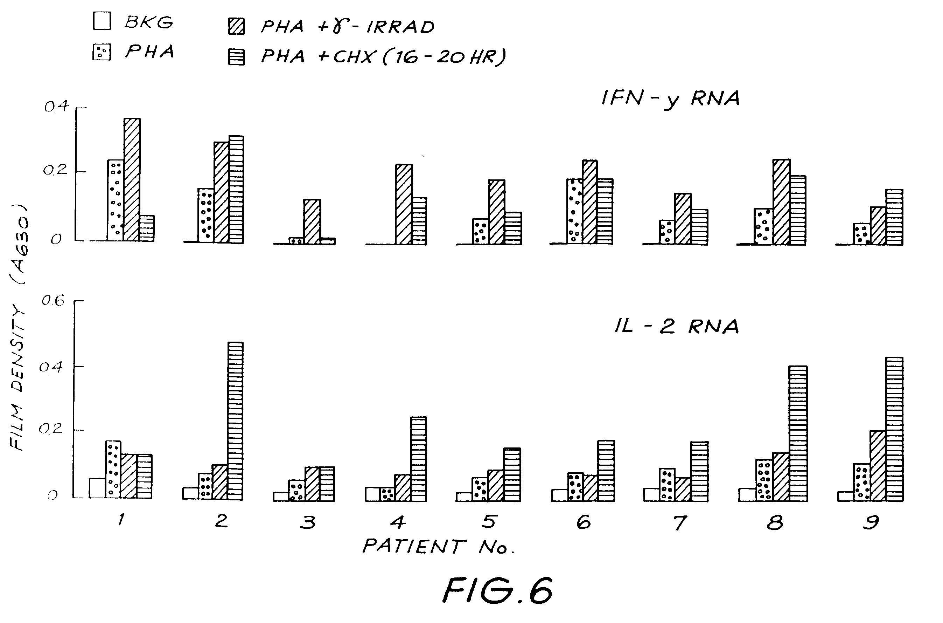

Fig. 5 is a graph showing the kinetics of IL-2 mRNA levels in human peripheral blood lymphocytes (PBL) induced with phytohemagglutinin (PHA). Cells were induced with PHA (0.4% v/v) at 0hr. Samples of 4 x 10⁶ cells were taken at each time point indicated, and RNA was extracted. Cycloheximide (CHX; 20ug/ml) was added at 21hr and RNA was analyzed at 24hr (▲).
Fig. 6 is a chart showing the quantitation of IL-2 and IFN-y gene expression in PBL of nine blood donors. 10ml of peripheral blood was taken from each donor. Cells from each donor were incubated with 1) no additions (background, BKG); 2) addition of 0.4% (v/v) PHA; 3) addition of PHA after Y-irradiation (1500 rad) of the cells; 4) addition of PHA, followed, at 16hr, by the addition of CHX (20ug/ml). RNA was extracted from all samples of 20hr after the addition of PHA and dot-blot filters were hybridized separately to IL-2 and IFN-y riboprobes. Bars denote film density for respective aasays, determining in a micro-elisa autoreader.

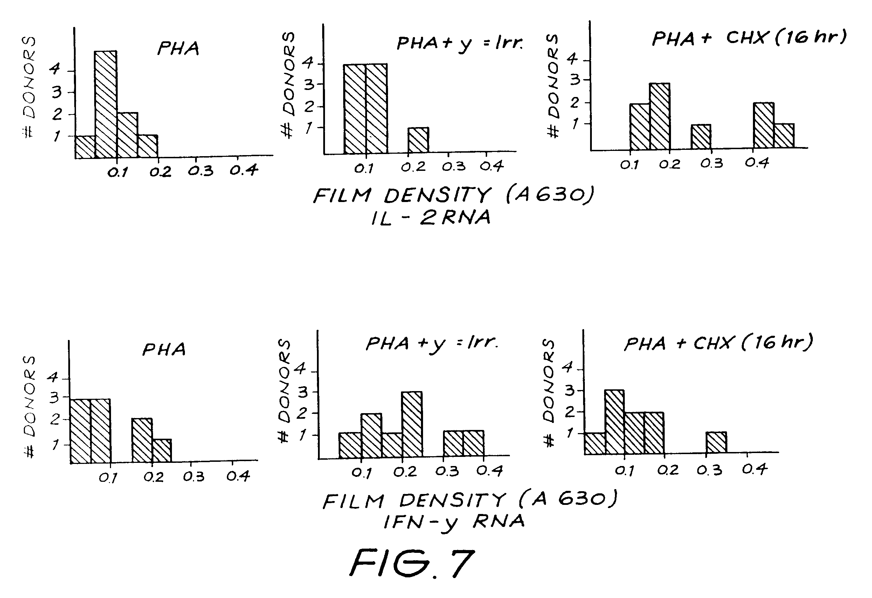

Fig. 7 is a chart showing the distribution of IL-2 and IFN-y gene expression levels in cells from the nine donors described in Fig. 6, expressed as absorbency of film at 630nm which reflects the amount of hybridized RNA.

A preferred method according to the present invention is as follows:

### Example 1 - Culture of Lymphocytes

1. Take 10ml of peripheral blood from patient in heparized tube.
2. Dilute 2-fold with phosphate-buffered saline (PBS).
3. Layer over 2vol Ficoll® (Pharmacia).
4. Spin 30 min at 200g at room temperature in cell centrifuge.
5. Wash twice with RPM1 1640 medium without serum.
6. Resuspend cells at a density of 4 x 10⁶ in 10-ml screw cap round-bottom plastic culture tubes (Cel-Cult) into RPM1 1640 medium containing:
   2 mM glutamine
   10 mM MEM non-essential amino acids
   100 mM MEM sodium pyruvate
   10 mM HEPES pH 7.2
   100 u/ml penicillin
   100 ug/ml streptomycin
   40 ug/ml gentamycin
   5 x 10⁻⁵ M 2-mercaptoethanol
   2% fetal calf serum
   5 ug/ml nystatin
7. Incubate overnight at 37°C in an atmosphere of 5% CO₂.

### Example 2 - Induction Conditions

Cells from each donor were incubated with 1) no additions (background, BKG): 2) addition of 0.4% (v/v) PHA; 3) addition of PHA after γ-irradiation (1500 rad) of the cells; 4) addition of PHA, followed, at 16hr, by the addition of CHX (20ug/ml). RNA was extracted from all samples at 20hr after the addition of PHA.

### Example 3 - Analytical Procedure. RNA Extraction

All steps are performed in a single microcentrifuge tube of 1.5ml, using a standard table-top microcentrifuge (Eppendorf).
1. Spin down 1ml of cell suspension in an Eppendorf centrifuge at 500rpm for 5 min.
2. Add 0.4ml of 7.5M guanidinium thiocyanate (GTC) may be used as described in Example 4.
3. Homogenize by vortexing the tube for at least 30 sec, until dissolved completely.
4. Add 20µl of 2 M KOAc pH 5.1. Mix and then add 250µl of absolute ethanol. Mix again.
5. Leave overnight at -20°C.
6. Centrifuge at 15,000 rpm for 30 min at 4°C in an Eppendorf centrifuge.
7. Discard supernatant.
8. Dissolve pellet into 50µl of 37% formaldehyde. Mix on a vortex until dissolved. Then add 50µl of 3 M NaCL, 0.3 M Na-citrate.
9. Place for 15 min at 60°C.
10. Perform a dot-block procedure. The sample is pipetted into a single well of a 96-place microtiter plate (Nunc) and serially diluted, in 2-fold steps, into 1.5 M NaCl, 0.15 M Na-citrate (10 x SSC) and pipetted into adjacent wells. After all samples have been diluted, the contents of each well of a 96-place manifold dot-blot device (Schleicher & Schuell) under vacuum, containing a 9 x 13cm nitrocellulose sheet (Schleicher & Schuell GB 003 blotting paper. The nitrocellulose sheet and the blotting paper were presoaked in distilled water and then in 10 x SSC. Each well is washed once with 100ul of 10 x SSC. The nitrocellulose is baked at 80°C for 2hr under vacuum and probed with the desired gene riboprobe, as described in examples 5 or 6.
11. Quantitate film dots in micro-elisa autoreader. To this end, exposed and developed X-ray film is cut into the shape of a microtiter plate, placed on the plate such that dots align with wells, and introduced into the autoreader (e.g., Dynatech®). Scanning is at a wavelength of 630nm.

### Example 4 - Alternative Procedure of RNA Extraction

1. Spin down 1ml of cell culture in an Eppendorf centrifuge at 500rpm for 5 min.
2. Discard supernatant and drain pellet.
3. Add 100µl of lysis buffer, consisting of 5 M GTC, 10 mM EDTA, 50 mM Tris-HCl pH 7.5, and 8% (v/v) 2-mercaptoethanol (added freshly).
4. Mix three times on vortex for 10 sec. Add 700ul of 4 M LiCl and leave for 20hr (overnight) at 4°C.
5. Spin in an Eppendorf centrifuge for 90 min at 12,000 rpm at 4°C.
6. Discard supernatant, dry pellet and 50µl of RNA solubilizing buffer: 0.1% sodium dodecyl sulfate (SDS), 1 mM EDTA, 10 mM Tris-HCl pH 7.5
7. Mix by vortexing for 20 sec. Repeat after every 10 min for 40 min. This step is done at room temperature.
8. Extract solution by vortexing it with an equal volume of phenol/chloroform (1:1), collect upper phase.
9. Add 1/10 vol of 3 M NaOAc pH 5.1, mix, add 2.5 vol absolute ethanol and leave at -20°C for 20 hr.
10. Precipitate RNA at 4°C by a 45 min centrifugation at 15,000 rpm in an Eppendorf centrifuge.
11. Wash pellet with 70% ethanol.
12. Discard supernatant and dry pellet.
13. Follow steps 8-11 of Example 3.

### Example 5 - Preparation of Riboprobe of IL-2

The riboprobe is synthesized using Promega-Biotec riboprobe kit. IL-2 cDNA was excised from plasmid p3-16 (Taniguchi T., Matsui, H., Fujita, T., Takaoka, C., Kashima, N., Yoshimoto, R., Hamuro, J. Nature 302 (1983) p.305) with Pst I and inserted into pGEM-3 (Promega Biotec)at the Pst I site. The orientation in which transcription from the T7 promoter gives the desired RNA strand was selected.
a) Transcript was made as follows:-
   1) 4µl transcript buffer.
   2) 2µl dithiotreitol (DDT) (100 mM).
   3) 0.6µl RNasin (350 u/ml).
   4) 1µl each of the nucleotides UTP, CTP, GTP (0.5 mM).
   5) 1µl T7 RNA polymerase (15 u/ml).
   6) 1µl plasmid containing the IL-2 cDNA (1 µg/1 ul.
   7) 10µl [α⁻³²P] ATP (10 mCi/ml).
   8) 10µl of [α-³⁵ ] ATP (10 mCi/ml) (Amersham) may be substituted for [α⁻³²P] ATP in the previous step.
b) The mixture is incubated for 1hr at 37°C.
c) 1ul DNase (free of RNase; 1u/ml is added and incubation is continued for 15 min at 37°C.
d) RNA is extracted as follows:
   1) 20µl of phenolchloroform (1:1) is added, followed by mixing on vortex and centrifugation for 2 min in an Eppendorf centrifuge.
   2) The upper (aqueous) phase is carefully collected.
   3) The lower phase of phenochloroform is washed with 20ul of TE buffer: 10 mM Tris-HCl pH 7.6, 1 mM EDTA.
   4) After vortexing and centrifugation of 2 min in an Eppendorf centrifuge, the upper phase is collected and added to the aqueous phase collected previously.
   5) 40µl of chloroform is added to the combined aqueous phases.
   6) After vortexing and centrifugation for 2 min in an Eppendorf centrifuge, the upper phase is collected.
   7) The lower phase is extracted with 40µl of TE buffer, as described under steps 3 and 4 above, and the aqueous phase is combined with the aqueous phase collected in step 6 above.
   8) 8µl of 3 M NaOAc pH 5.1 and 2.5 vol of absolute ethanol are added to the combined aqueous phases.
   9) The solution is stored for 20 hr (overnight) at -20°C, or for 2 hr at -70°C.
   10) Centrifuge for 30 min at 4°C in an Eppendorf centrifuge at 15,000 rpm.
   11) Wash pellet with 1ml of 70% ethanol.
   12) Dry pellet in vacuo.
   13) Resuspend pellet in 100µl TEN buffer containing: 10 mM Tris-HCl, pH 7.6, 1 mM EDTA, 0.1 M Na Cl and 20 ug/ml tRNA from E.coli MRE 600 (Boehringer).
   14) Apply to a 1-ml Sephadex® G-50 column made in a 2-ml syringe and equilibrated with TEN buffer.
   15) Centrifuge the column for 5 min at 1,000 rpm in a cell centrifuge and collect the flow-through in a tube.
   16) Take a 1ul sample into 10% ice-cold trichloroacetic acid and determine precipitable radioactivity.
   17) A good preparation yields 3 - 10 x 10⁵ cpm/µl.

### Example 6 - Preparation of Riboprobe for IFN-y

The riboprobe for IFN-y gene expression was synthesized following the procedure described in Example 5. IFN-y cDNA was excised from plasmid pH11F-SV-y with Bam H1 (Devos, R., Cheroutre, H., Taya, Y., Degrave, W., Heuverswyn, H.Y., Fiers, W. Nucl. Acids Res. 10 (1982) p.2487) and inserted into pGEM-3 at the Bam H1 site. The orientation in which transcription from the T7 promotor gives the desired RNA strand was selected.

### Example 7 - Hybridization of Nitrocellulose Filter

1) Filters are prehybridized in a sealed plastic bag for 4 hr at 60°C. The prehybridization buffer contains:
   25% formamide
   10% dextran sulfate (5,000 mol weight)
   1% SDS
   50 mM Tris-HCl pH 7.5
   100 µg/ml salmon sperm DNA that has been boiled for
   10 min and cooled rapidly
   100 µg/ml tRNA (E.coli MRE 600)
   0.7 M NaCl
2) For hybridization add: 5 x 10⁶ cpm/ml of riboprobe (see above) x 1
   Denhardt solution: 0.02% (w/v) Ficoll, 0.02% (w/v)
   polyvinyl-pyrrolidone, 0.02% (w/v) BSA fraction V
   (Sigma)
   100 µg/ml tRNA (E.coli MRE600)
   Incubate for 16-18 hr, but not more, at 60°C.
3) After hybridization, filters are washed:
   1) twice with 2 x SSC (500 ml) at room temperature for 15 min
   2) twice with 2 x SSC, 1% SDS (500 ml) at 68°C for 30 min
   3) once with 0.2 x SSC, 0.2% SDS (500 ml) at 68°C for 30 min.

Filters are dried and exposed to X-ray film for 5-25 hr at -70°C.

### ANALYSIS OF RESULTS

Results may be analyzed as follows:

| | IL-2 | IFN-Y |
|---|---|---|
| No inducer | 1A | 1B |
| PHA (16 hr) | 2A | 2B |
| PHA (20 hr) | 3A | 3B |
| PHA + cycloheximide | 4A | 4B |
| PHA + Y-irradiation | 5A | 5B |

(a) Basal level of gene expression:
   1A, 1B.
(b) Inducibility of genes
   Ratio 2A/1A, 2B/1B. Ratio 3A/1A, 3B/1B.
   The difference between samples 2 and 3 is kinetic: transient expression of these genes may peak by 16 hr or 20 hr, depending on the donor. Normally, these values should be 3-10; if less, this means either that the genes are highly activated, or that they fail to respond to induction. These possibilities can be distinguished in step (c) below.
(c) Superinducibility by cycloheximide:
   A4/A3, B4/B3.
   The ratio shows the potential for gene expression. Normally, this ratio should be considerably greater than 1 (1.5-5 or more). If not, this means that either basal expression is very high, in which case the ratios A4/A1, B4/B1 should be close to 1, or, alternatively, that induced gene expression is very high and uncontrolled, in which case the ratios A3/A1 and B3/B1 should be high.
(d) Superinducibility by Y-irradiation:
   A5/A3, B5/B3.
   Normally, this ratio should be well in excess of 1 (1.5-3). Normally, the ratio B5/B3 will exceed the ratio A5/A3. These values measure the extent to which suppressor T cells down-regulate IL-2 and IFN-Y gene expression; the latter gene is more strongly regulated in this manner. The higher these ratios, the stronger the down-regulation.

## Claims

1. A method for calculating the inductivity of a bioregulatory gene in a sample of eukaryotic cells, the method comprising:
a) isolating a sample of eukaryotic cells;
b) dividing the sample into at least two aliquots;
c) culturing the cells in each aliquot;
d) lysing the cultured cells in each aliquot by the addition of guanidinium thiocyanate or guanidine hydrochloride lysing agent;
e) homogenising each aliquot and thereafter selectively precipitating the RNA in each aliquot by the successive addition of sodium or potassium acetate and absolute ethanol;
f) recovering and blotting the RNA from each of aliquot onto a support matrix;
g) hybridising the recovered and blotted RNA from each aliquot with a labelled riboprobe specific to the bioregulatory gene; and
h) quantitatively determining the extent of expression of the bioregulatory gene by measuring the extent of binding of the labelled riboprobe to the blotted RNA;
the method being characterised in that in step (c):
(c) (i) the cells in a first aliquot are cultured in the presence of an inducing agent for inducing expression of the bioregulatory gene; and
(c) (ii) the cells in a second aliquot are cultured in the absence of an inducing agent;
and in that:
i) the ratio of the extent of gene expression in the first aliquot to the extent of gene expression in the second aliquot is calculated, said ratio providing a measure of the inductivity of the bioregulatory gene.

2. A method as claimed in Claim 1 wherein said sample of eukaryotic cells is a sample of cells selected from peripheral human blood mononuclear cells, lymphocytes, monocytes, fibroblasts, astrocytes, endothelial cells, glial cells, keratinocytes and platelets.

3. A method as claimed in Claim 1 or Claim 2 wherein said inducing agent is phytohemagglutinin (PHA) or concavalin A.

4. A method as claimed in Claim 1, Claim 2 or Claim 3 characterised in that the cells in a third aliquot are superinduced; and in that the ratio of the extent of gene expression in the third aliquot to the extent of gene expression in the first aliquot is calculated to provide a measure of the maximum extent of expression of the bioregulatory gene.

5. A method as claimed in Claim 4 wherein the cells in the third aliquot are cultured in the presence of a superinducing agent selected fom cycloheximide, cycloheximide + phytohemagglutinin and cycloheximide + concavalin A.

6. A method as claimed in Claim 4 wherein the cells in the third aliquot are cultured in the presence of an inducing agent selected from phytohemagglutinin and concavalin A, and prior to adding said inducing agent are irradiated with gamma-radiation to superinduce said cells.

7. A method as claimed in any preceding claim wherein in step (g) the riboprobe is specific to a bioregulatory gene selected from the genes coding for interferons, interleukins, tumour necrosis factors and growth factors.

8. A method as claimed in any preceding claim wherein in step (g) the riboprobe is specific to a bioregulatory gene coding for IL-2 or IFN-y.

9. A method as claimed in any preceding claim wherein step (e) lithium chloride and guanidinium thiocyanate are added to each aliquot prior to the sodium or potassium acetate.

10. A method as claimed in any preceding claim wherein the riboprobe is labelled with a detectable isotopic or non-isotopic chemical group.

11. A method as claimed in claim 10 wherein said riboprobe is isotopically labelled, and in step (h) the extent of binding is measured by measuring the absorbancy of a photographic film blackened by exposure to the support matrix carrying the RNA and hybridized labelled riboprobe.

12. A method of evaluating the dynamic functionality of the immune response in a sample of eukaryotic cells, the method comprising a method as claimed in any of Claims 1 - 11 using said sample.

13. An in vitro diagnostic method for diagnosing viral, bacterial, fungal, oncogenic and immunoregulatory diseases in a patient, the diagnostic method comprising a method as claimed in any of Claims 1 - 11 using a sample of blood from the patient.

## Patentansprüche

1. Verfahren zur Berechnung der Induzierbarkeit eines bioregulatorischen Gens in einer Probe von eukaryotischen Zellen, das folgende Schritte umfaßt:
a) Isolieren einer Probe der eukaryotischen Zellen,
b) Aufteilen der Probe in mindestens zwei Aliquote,
c) Kultivieren der Zellen in jedem Aliquot,
d) Lysieren der kultivierten Zellen in jedem Aliquot durch Zugabe von Guanidiniumthiocyanat oder Guanidinhydrochlorid als lysierendes Mittel,
e) Homogenisieren jedes Aliquots und darauffolgend selektives Fällen der RNA in jedem Aliquot durch aufeinanderfolgende Zugabe von Natrium- oder Kaliumacetat und absolutem Ethanol,
f) Gewinnen und Blotting der RNA aus jedem Aliquot auf eine Trägermatrix,
g) Hybridisieren der aus jedem Aliquot gewonnenen und durch Blotting übertragenen RNA mit einer markierten, für das bioregulatorische Gen spezifischen RNA-Sonde und
h) quantitative Bestimmung des Ausmaßes der Expression des bioregulatorischen Gens durch Messen des Ausmaßes der Bindung der markierten RNA-Sonde an die übertragene RNA,
wobei das Verfahren dadurch gekennzeichnet ist, daß in Schritt c):
(c) (i) die Zellen in dem ersten Aliquot in Gegenwart eines Mittels kultiviert werden, das die Expression des bioregulatorischen Gens induziert, und
(c) (ii) die Zellen in dem zweiten Aliquot ohne ein induzierendes Mittel kultiviert werden,
und daß
i) das Verhältnis des Ausmaßes der Genexpression im ersten Aliquot zum Ausmaß der Genexpression im zweiten Aliquot berechnet wird, wobei dieses Verhältnis ein Maß für die Induzierbarkeit des bioregulatorischen Gens liefert.

2. Verfahren nach Anspruch 1, wobei die Probe der eukaryotischen Zellen eine Probe von Zellen ist, die unter einkernigen Zellen des peripheren menschlichen Blutes, Lymphocyten, Monocyten, Fibroblasten, Astrocyten, Endothelzellen, Gliazellen, Keratinocyten und Blutplättchen ausgewählt sind.

3. Verfahren nach Anspruch 1 oder 2, wobei das induzierende Mittel Phytohämagglutinin (PHA) oder Concanavalin A ist.

4. Verfahren nach Anspruch 1, 2 oder 3, dadurch gekennzeichnet, daß die Zellen in einem dritten Aliquot superinduziert werden und das Verhältnis des Ausmaßes der Genexpression in dem dritten Aliquot zum Ausmaß der Genexpression in dem ersten Aliquot berechnet wird, womit ein Maß für das maximale Ausmaß der Expression des bioregulatorischen Gens geliefert wird.

5. Verfahren nach Anspruch 4, wobei die Zellen in dem dritten Aliquot in Gegenwart eines superinduzierenden Mittels, das unter Cycloheximid, Cycloheximid + Phytohämagglutinin und Cycloheximid + Concanavalin A ausgewählt ist, kultiviert werden.

6. Verfahren nach Anspruch 4, wobei die Zellen in dem dritten Aliquot in Gegenwart eines induzierenden Mittels, das unter Phytohämagglutinin und Concanavalin A ausgewählt ist, kultiviert werden und bei dem die Zellen vor der Zugabe des induzierenden Mittels zur Superinduktion bei diesen Zellen mit γ-Strahlen bestrahlt werden.

7. Verfahren nach einem der vorhergehenden Ansprüche, wobei die RNA-Sonde in Schritt (g) für ein bioregulatorisches Gen spezifisch ist, das unter den Genen ausgewählt sind, die Interferone, Interleukine, Tumornekrosefaktoren und Wachstumsfaktoren codieren.

8. Verfahren nach einem der vorhergehenden Ansprüche, wobei die RNA-Sonde in Schritt (g) spezifisch ist für ein bioregulatorisches Gen, das IL-2 oder IFNγ codiert.

9. Verfahren nach einem der vorhergehenden Ansprüche, wobei in Schritt (e) jedem Aliquot vor der Zugabe von Natrium- oder Kaliumacetat Lithiumchlorid und Guanidiniumthiocyanat zugegeben werden.

10. Verfahren nach einem der vorhergehenden Ansprüche, wobei die RNA-Sonde mit einer nachweisbaren chemischen Gruppe markiert ist, die gegebenenfalls ein Isotop enthält.

11. Verfahren nach Anspruch 10, wobei die RNA-Sonde mit einem Isotop markiert ist und in Schritt (h) das Ausmaß der Bindung durch Messen des Absorptionsvermögens eines photographischen Films gemessen wird, der dadurch geschwärzt wurde, daß er der Trägermatrix ausgesetzt wurde, die die RNA und die hybridisierte, markierte RNA-Sonde trägt.

12. Verfahren zur Ermittlung der dynamischen Funktionalität der Immunantwort in einer Probe eukaryotischer Zellen, das ein Verfahren nach einem der Ansprüche 1 bis 11 einschließt, bei dem diese Probe verwendet wird.

13. In-vitro-Diagnoseverfahren zur Diagnose von viralen, bakteriellen, pilzbedingten, onkogenen und immunregulatorischen Erkrankungen bei Patienten, das ein Verfahren nach einem der Ansprüche 1 bis 11 einschließt, bei dem eine Blutprobe des Patienten verwendet wird.

## Revendications

1. Méthode pour calculer l'aptitude à l'induction d'un gène biorégulateur dans un échantillon de cellules eucaryotes, la méthode comprenant:
a) l'isolement d'un échantillon de cellules eucaryotes;
b) la division de l'échantillon en au moins deux portions;
c) la culture des cellules dans chaque portion;
d) la lyse des cellules cultivées dans chaque portion par addition de thiocyanate de guanidinium ou de chlorhydrate de guanidinium comme agent de lyse;
e) l'homogénéisation de chaque portion puis la précipitation sélective de l'ARN dans chaque portion par addition successive d'acétate de sodium ou de potassium et d'éthanol absolu;
f) la récupération et le transfert de l'ARN à partir de chaque portion sur une matrice support;
g) l'hybridation de l'ARN récupéré et transféré de chaque portion avec une sonde ribonucléique marquée spécifique du gène biorégulateur; et
h) la détermination quantitative du degré d'expression du gène biorégulateur par la mesure du taux de liaison de la sonde ribonucléique marquée à l'ARN transféré;
la méthode étant caractérisée en ce que, dans l'étape (c):
(c) (i) on cultive les cellules d'une première portion en présence d'un agent inducteur pour l'induction de l'expression du gène biorégulateur; et
(c) (ii) on cultive les cellules d'une seconde portion en l'absence d'agent inducteur;
et en ce que
i) on calcule le rapport du degré d'expression du gène dans la première portion au degré d'expression du gène dans la seconde portion, ledit rapport donnant une mesure de l'aptitude à l'induction du gène biorégulateur.

2. Méthode selon la revendication 1, dans laquelle ledit échantillon de cellules eucaryotes est un échantillon de cellules choisies parmi des cellules mononucléaires de sang périphérique humain, des lymphocytes, des monocytes, des fibroblastes, des astrocytes, des cellules endothéliales, des cellules gliales, des kératinocytes et des plaquettes.

3. Méthode selon la revendication 1 ou la revendication 2, dans laquelle ledit agent inducteur est la phytohémagglutinine (PHA) ou la concavaline A.

4. Méthode selon la revendication 1, la revendication 2 ou la revendication 3, caractérisée en ce que l'on soumet les cellules d'une troisième portion à une superinduction; et en ce que l'on calcule le rapport du degré d'expression du gène dans la troisième portion au degré d'expression du gène dans la première portion pour obtenir une mesure du degré maximal d'expression du gène biorégulateur.

5. Méthode selon la revendication 4, dans laquelle les cellules de la troisième portion sont cultivées en présence d'un agent superinducteur choisi parmi le cycloheximide, le cycloheximide + la phytohémagglutinine et le cycloheximide + la concavaline A.

6. Méthode selon la revendication 4, dans laquelle on cultive les cellules de la troisième portion en présence d'un agent inducteur choisi parmi la phytohémagglutinine et la concavaline A, et en ce que, avant d'ajouter ledit agent inducteur, on les irradie avec un rayonnement gamma pour effectuer une superinduetion desdites cellules.

7. Méthode selon l'une quelconque des revendications précédentes, dans laquelle, dans l'étape (g), la sonde ribonucléique est spécifique d'un gène biorégulateur choisi parmi les gènes codant pour les interférons, les interleukines, les facteurs de nécrose des tumeurs et les facteurs de croissance.

8. Méthode selon l'une quelconque des revendications précédentes, dans laquelle, dans l'étape (g), la sonde ribonucléique est spécifique d'un gène biorégulateur codant pour l'IL-2 ou l'IFN-γ.

9. Méthode selon l'une quelconque des revendications précédentes, dans laquelle, dans l'étape (e), on ajoute du chlorure de lithium et du thiocyanate de guanidinium à chaque portion avant d'ajouter l'acétate de sodium ou de potassium.

10. Méthode selon l'une quelconque des revendications précédentes, dans laquelle la sonde ribonucléique est marquée avec un groupe chimique isotopique ou non isotopique décelable.

11. Méthode selon la revendication 10, dans laquelle ladite sonde ribonucléique est marquée avec un isotope, et, dans l'étape (h), on mesure le taux de liaison en mesurant l'absorbance d'un film photographique noirci par l'exposition à la matrice support portant l'ARN et la sonde ribonucléique marquée hybridée.

12. Méthode d'évaluation de la fonctionnalité dynamique de la réponse immunitaire dans un échantillon de cellules eucaryotes, la méthode comprenant une méthode selon l'une quelconque des revendications 1 à 11 utilisant ledit échantillon.

13. Méthode de diagnostic *in vitro* pour le diagnostic de maladies virales, bactériennes, fongiques, oneogéniques et du système immunorégulateur chez un patient, la méthode de diagnostic comprenant une méthode selon l'une quelconque des revendications 1 à 11 utilisant un échantillon de sang du patient.
